(19) Europäisches Patentamt
European Patent Office
Office européen des brevets

(11) Numéro de publication: **0 133 078**
**A1**

(12) # DEMANDE DE BREVET EUROPEEN

(21) Numéro de dépôt: **84401407.6**

(22) Date de dépôt: **03.07.84**

(51) Int. Cl.⁴: **C 08 B 37/10**

(30) Priorité: **04.07.83 FR 8311052**

(43) Date de publication de la demande:
**13.02.85 Bulletin 85/7**

(84) Etats contractants désignés:
**AT BE CH DE FR GB IT LI LU NL SE**

(71) Demandeur: **RHONE-POULENC SANTE**
**Les Miroirs 18 Avenue d'Alsace**
**F-92400 Courbevoie Cedex(FR)**

(72) Inventeur: **Mardiguian, Jean Sarkis**
**3, Villa Christine**
**F-94310 La Varenne-St-Hilaire(FR)**

(74) Mandataire: **Gaumont, Robert et al,**
**RHONE-POULENC RECHERCHES Service Brevets**
**Pharma 25, Quai Paul Doumer**
**F-92408 Courbevoie Cedex(FR)**

(54) **Oligosaccharides sulfatés, leur procédé de préparation et leur utilisation comme médicaments.**

(57) Composés, utiles comme médicaments, de formule:

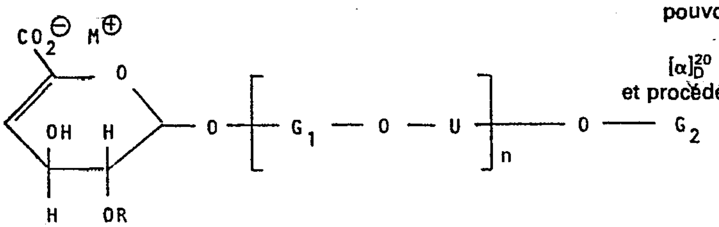
(I)

dans laquelle n est 2 à 9, U est un chaînon du type acide uronique, $G_1$ et $G_2$ sont des chaînons de formules respectives:

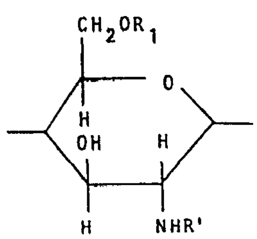
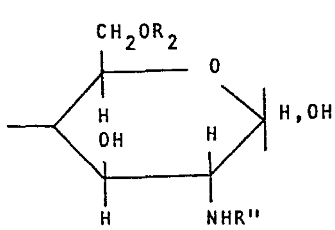

R, $R_1$ et $R_2$ sont H ou $SO_3^-$ $M^+$, R' et R'' sont $SO_3^-$ $M^+$ ou $CH_3-CO-$, $-O-$ est un pont oxygène et $M^+$ est un cation métallique, lesdits composés présentant en outre les caractéristiques suivantes:
pourcentage de soufre: 5 % à 13,5 %
pourcentage d'azote : 1,8 % à 2,5 %
pourcentage en poids de groupes acétyle : 0 % à 8 %
pouvoir rotatoire spécifique en solution aqueuse à 20°C:

$[\alpha]_D^{20}$ = + 30° à + 70°

et procédé pour leur préparation.

EP 0 133 078 A1

La présente invention a pour objet des oligosaccharides sulfatés utilisables comme médicaments, en particulier comme médicaments antithrombotiques et hypolipémiants et leur procédé de préparation.

Les oligosaccharides sulfatés selon l'invention répondent à la formule :

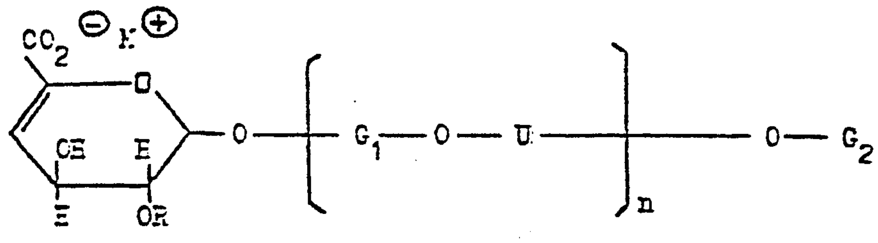

(I)

dans laquelle n est un nombre entier de 2 à 9 , les chaînons U de la chaîne $-[G_1-O-U]_n-$ sont des chaînons du type acide uronique (acide D-glucuronique, acide L-iduronique ou acide L-iduronique sulfaté en position 2), les chaînons $G_1$ de la chaîne $-[G_1-O-U]_n-$ répondent à la formule :

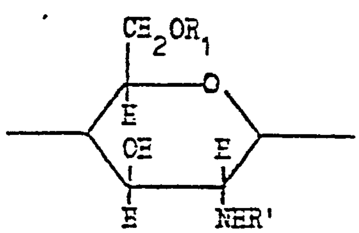

$G_2$ est un chaînon de formule :

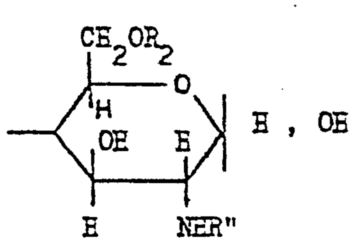

R, $R_1$ et $R_2$ désignent des atomes d'hydrogène ou des groupes $SO_3^{\ominus} M^{\oplus}$, R' et R" désignent des groupes $SO_3^{\ominus} M^{\oplus}$ ou $CH_3-\underset{\underset{O}{\|}}{C}-$, -O- désigne un pont oxygène et $M^{\oplus}$ désigne un cation métallique monovalent tel que $Na^+$ ou la moitié d'un cation métallique bivalent tel que $Ca^{2+}$, $Mg^{2+}$ ou $Cu^{2+}$.

Les oligosaccharides sulfatés selon l'invention présentent en outre les caractéristiques suivantes :

pourcentage de soufre : 5 % à 13,5 %
pourcentage d'azote : 1,8 % à 2,5 %
pourcentage en poids de groupes acétyle : 0 % à 8 %
pouvoir rotatoire spécifique en solution aqueuse à 20°C:

$$[\alpha]_D^{20} = + 30^\circ \text{ à } + 70^\circ$$

Les oligosaccharides préférés sont ceux dans lesquels le pourcentage de soufre est de 5 % à 8 % et le pourcentage en poids de groupes acétyle est de 0 % à 6 %.

Les oligosaccharides sulfatés selon l'invention peuvent être préparés, à partir d'héparine ou d'héparine partiellement désulfatée, par le procédé en trois étapes suivant :

1°) On prépare un ester d'héparine ou d'héparine partiellement désulfatée dans lequel sont estérifiés, partiellement ou totalement, uniquement les groupes acide carboxylique de l'héparine ou de l'héparine partiellement désulfatée.

2°) On soumet l'ester ci-dessus à l'action d'une base minérale ou organique telle que l'hydroxyde de sodium, l'hydroxyde de potassium, un carbonate alcalin, la triéthylamine, la triéthylénediamine, la quinuclidine, le diaza-1,5 bicyclo [4.3.0] nonène-5 ou le diaza-1,5 bicyclo [5.4.0] undecène -5, en milieu aqueux ou au sein d'un solvant organique inerte, à une température située dans l'intervalle 20°C-80°C, la concentration molaire de la base dans le milieu étant de préférence située dans l'intervalle 0,1-0,6. L'action de la base sur l'ester permet de réaliser une dépolymérisation partielle et contrôlée de l'héparine ou de l'héparine partiellement désulfatée sans altérer sa structure générale.

3°) Le produit de dépolymérisation obtenu dans la deuxième étape, qui est un mélange de polysaccharides sulfatés ayant un poids moléculaire moyen en poids inférieur à celui de l'héparine ou de l'héparine partiellement désulfatée de départ, est fractionné sur une colonne de gel polyacrylamide-agarose comprenant 20 % d'acrylamide et 2 % d'agarose en utilisant comme éluant une solution aqueuse molaire de chlorure de sodium.

La première étape du procédé est décrite en particulier dans les brevets anglais 973 894 et 1 501 095, le brevet français 2 150 724, le brevet français de médicament 2739.M et la demande de brevet européen n° 81 400 727.4.

La deuxième étape du procédé est décrite en particulier dans la demande de brevet européen n° 81 400 728.2.

Les oligosaccharides sulfatés selon l'invention présentent des activités antithrombotique (sans effet hémorragique), fibrinolytique, anti-inflammatoire et hypolipémiante. Ils sont utilisables en particulier pour la prévention des thromboses post-opératoires et le traitement des thromboses constituées, et pour le traitement de l'hyperlipémie.

Ils peuvent être administrés, en mélange avec un véhicule pharmaceutiquement acceptable, par voie intraveineuse, par voie sous-cutanée, par voie orale ou par voie pulmonaire (inhalation).

La posologie dépend des effets recherchés et de la voie d'administration utilisée. Par exemple, par voie sous-cutanée elle est de 1 à 5 mg/kg/jour. Administrés par voie sous-cutanée, les oligosaccharides selon l'invention présentent une longue durée d'action.

Ces produits sont atoxiques à 100 mg/kg par voie orale et 10 mg/kg par voie intraveineuse.

Les exemples suivants illustrent l'invention sans la limiter.

Exemples 1 à 9 :

1°) Préparation de l'ester d'héparine :

75 g d'héparinate de benzéthonium, provenant d'une héparine d'intestin de porc ayant un poids moléculaire moyen en poids de 12 000 à 16 000 daltons et conforme aux normes de la Pharmacopée Française, 8ème édition, sont traités par 75 ml de chlorure de benzyle, au sein de 375 ml de dichlorométhane, pendant 72 heures à 25°C.

On ajoute ensuite au milieu réactionnel 375 ml d'une solution à 10 % d'acétate de sodium dans le méthanol. Le précipité formé est séparé par filtration, lavé au méthanol, puis à l'acétone, et séché sous vide à la température ambiante. On obtient ainsi 30,9 g d'ester benzylique brut d'héparine, sous forme de sel de sodium.

Les 30,9 g d'ester benzylique brut d'héparine, sous forme de sel de sodium, sont dissous dans 300 ml d'eau, et on ajoute du chlorure de sodium jusqu'à ce que la concentration en NaCl du milieu atteigne 10 %. On ajoute alors 750 ml de méthanol. Le précipité formé est isolé par filtration, lavé au méthanol puis à l'acétone, et séché sous vide à la température ambiante. On obtient ainsi 27, 21 g d'ester benzylique purifié d'héparine, sous forme de sel de sodium.

2°) Dépolymérisation :

Les 27, 21 g d'ester benzylique purifié ci-dessus sont traités pendant deux heures, à 60°C, par 650 ml d'une solution aqueuse 0,1 N d'hydroxyde de sodium. La solution est ensuite refroidie et neutralisée par addition d'une solution aqueuse N d'acide chlorhydrique. On ajoute ensuite 68 g de chlorure de sodium NaCl, puis 1 300 ml de méthanol. Le précipité formé est isolé par filtration, lavé au méthanol puis à l'acétone, et séché sous vide à 50°C. On obtient ainsi 19 g d'héparine dépolymérisée brute, sous forme de sel de sodium.

Les 19 g d'héparine dépolymérisée brute ci-dessus sont dissous dans 190 ml d'eau, et on ajoute 1,90 ml d'eau oxygénée à 110 volumes. On laisse 72 heures en contact, puis on filtre et ajuste la concentration en NaCl du filtrat à 10 % par addition de chlorure de sodium.

On ajoute ensuite au filtrat 475 ml de méthanol. Le précipité formé est isolé par filtration, lavé au méthanol puis à l'acétone, et séché sous vide à 50°C. On obtient ainsi 18 g d'héparine dépolymérisée, sous forme de sel de sodium.

3°) Fractionnement sur gel polyacrylamide-agarose :

On utilise une colonne de verre de diamètre 50 mm et de hauteur 2 mètres, contenant 3,6 litres du gel polyacrylamide-agarose connu sous la dénomination Ultrogel AcA 202 (gel, sous forme de perles ayant un diamètre de 60 à 140 µm, contenant 20 % d'acrylamide, 2 % d'agarose et un agent réticulant).

Une prise de 2 g d'héparine dépolymérisée (sous forme d'une solution dans l'éluant) est placée en tête de colonne, et on élue par une solution aqueuse 1 M de chlorure de sodium, à la vitesse de 50 ml par heure. On recueille en bas de colonne des fractions de 20 ml et on mesure l'absorption à 230 nm de chaque fraction. On trace la courbe d'élution (courbe donnant la valeur de l'absorption à 230 nm

en fonction du numéro de la fraction), et on regroupe les fractions correspondant à un même pic d'élution.
Les fractions regroupées sont concentrées sous vide, à 40°C, jusqu'à ce que leur teneur en NaCl atteigne 10 %. On ajoute alors 4 volumes de méthanol (c'est-à-dire quatre fois le volume de la phase aqueuse). Le précipité formé est séparé par filtration, lavé à l'acétone, puis séché sous vide à 50°C.

On réalise la même opération successivement sur six prises de 2 g d'héparine dépolymérisée, et on isole ainsi les oligosaccharides sulfatés dont les propriétés sont rassemblées dans le tableau ci-dessous.

— Dans ce tableau, "absorption UV" désigne l'absorption à 230 nm, sous une épaisseur de 1 cm, d'une solution à 1 % du produit, et "viscosité" désigne la viscosité d'une solution aqueuse à 10 % du produit.

Les activités anti-Xa et A.P.T.T. ont été déterminées par la méthode de TEIEN et al., Thrombosis Research, 11, 107, 1977 et sont exprimées en unités internationales par mg (u.i./mg) en se référant au 3ème étalon international.

| Exemple | Poids de produit isolé | Valeur de n dans la formule (I) | Nom | $[\alpha]^{20}_{D}$ | Absorption UV | Viscosité (centi-poises) | Activité anti-Xa (u.i./mg) | Activité APTT (u.i./mg) |
|---|---|---|---|---|---|---|---|---|
| 1 | 0,060 g | 1 | Tétrasaccharide | | 44 | | | |
| 2 | 0,45 g | 2 | Hexasaccharide | | 29,2 | | 40 | 10 |
| 3 | 0,77 g | 3 | Octosaccharide | + 32° 6 | 21,4 | 1,27 | 68 | 12 |
| 4 | 0,81 g | 4 | Décasaccharide | + 38° 6 | 18,5 | 1,37 | 100 | 16 |
| 5 | 0,84 g | 5 | Dodécasaccharide | + 41° 4 | 14,8 | 1,47 | 110 | 18 |
| 6 | 0,81 g | 6 | Tétradécasaccharide | + 43° 8 | 12,7 | 1,56 | 130 | 20 |
| 7 | 0,70 g | 7 | Hexadécasaccharide | + 48° | 11,3 | 1,65 | 158 | 22 |
| 8 | 0,54 g | 8 | Octodécasaccharide | + 47° 6 | 10,0 | 1,82 | 158 | 22 |
| 9 | 0,24 g | 9 | Eicosaccharide | + 48° 4 | 9 | | 165 | 26 |

# R E V E N D I C A T I O N S

1 - Oligosaccharides sulfatés de formule :

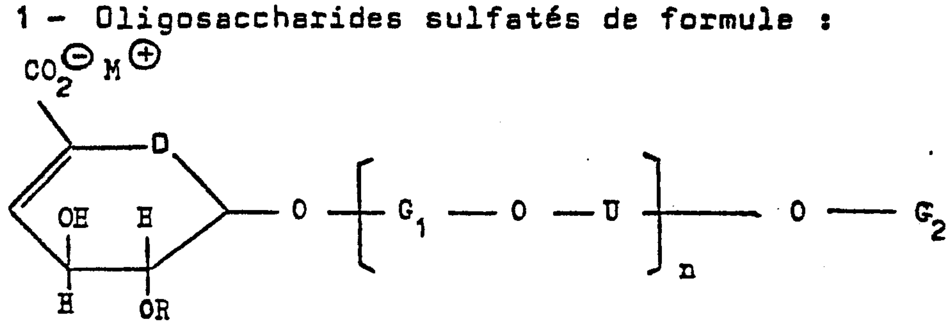

(I)

dans laquelle n est un nombre entier de 2 à 9, les chaînons U de la chaîne $\left[G_1-O-U\right]_n$ sont des chaînons du type acide uronique (acide D-glucuronique, acide L-iduronique ou acide L-iduronique sulfaté en position 2), les chaînons $G_1$ de la chaîne $\left[G_1-O-U\right]_n$ répondent à la formule :

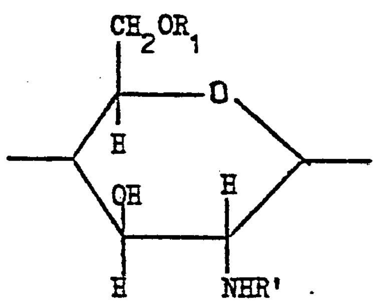

$G_2$ est un chaînon de formule :

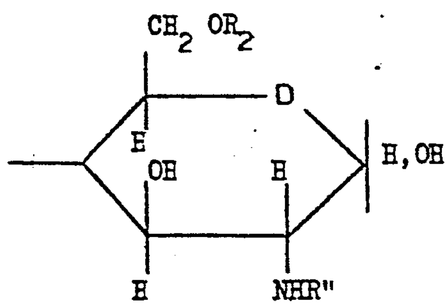

R, $R_1$ et $R_2$ désignent des atomes d'hydrogène ou des groupes $SO_3^{\ominus} M^{\oplus}$, R' et R" désignent des groupes $SO_3^{\ominus} M^{\oplus}$ ou $CH_3-\underset{\underset{O}{\|}}{C}-$, -O- désigne un pont oxygène et $M^{\oplus}$ désigne un cation métallique monovalent ou la moitié d'un cation métallique bivalent, lesdits oligosaccharides sulfatés présentant en outre les caractéristiques suivantes :

pourcentage de soufre : 5 % à 13,5 %
pourcentage d'azote : 1,8 % à 2,5 %
pourcentage en poids de groupes acétyle : 0 % à 8 %
pouvoir rotatoire spécifique en solution aqueuse à 20°C :

$$[\alpha]_D^{20} = +30° \text{ à } +70°$$

2 - Oligosaccharides sulfatés selon la revendication 1 dans lesquels le pourcentage de soufre est de 5 à 8 % et le pourcentage en poids de groupes acétyle est de 0 à 6 %.

3 - Oligosaccharides sulfatés selon la revendication 1 ou 2 dans lesquels $M^{\oplus}$ désigne $Na^{\oplus}$ ou la moitié d'un cation métallique bivalent choisi parmi $Ca^{2+}$, $Mg^{2+}$ et $Cu^{2+}$.

4 - Procédé de préparation des oligosaccharides sulfatés selon la revendication 1 caractérisé en ce que l'on fractionne sur gel de polyacrylamide-agarose comprenant 20 % de polyacrylamide et 2 % d'agarose, une héparine dépolymérisée éventuellement partiellement désulfatée obtenue par action d'une base minérale ou organique sur un ester d'héparine ou d'héparine partiellement désulfatée.

5 - Médicaments, caractérisés en ce qu'ils contiennent un oligosaccharide sulfaté selon l'une quelconque des revendications 1 à 3 associé à un véhicule pharmaceutiquement acceptable.

## REVENDICATION

1 - Procédé de préparation d'oligosaccharides de formule :

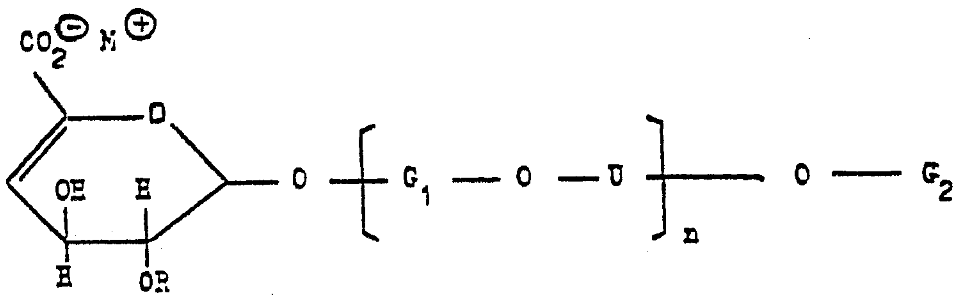

(I)

dans laquelle n est un nombre entier de 2 à 9, les chaînons U de la chaîne $-[G_1-O-U]_n-$ sont des chaînons du type acide uronique (acide D-glucuronique, acide L-iduronique ou acide L-iduronique sulfaté en position 2), les chaînons $G_1$ de la chaîne $-[G_1-O-U]_n-$ répondent à la formule :

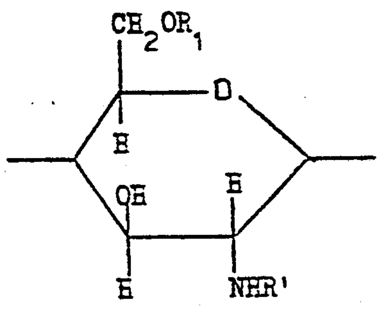

$G_2$ est un chaînon de formule :

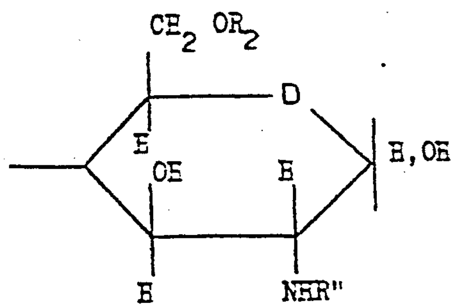

R, $R_1$ et $R_2$ désignent des atomes d'hydrogène ou des groupes $SO_3^{\ominus}M^{\oplus}$, R' et R" désignent des groupes $SO_3^{\ominus}M^{\oplus}$ ou $CH_3-\underset{\underset{O}{\|}}{C}-$, -O- désigne un pont oxygène et $M^{\oplus}$ désigne un cation métallique monovalent ou la moitié d'un cation métallique bivalent, lesdits oligosaccharides sulfatés présentant en outre les caractéristiques suivantes :

pourcentage de soufre : 5 % à 13,5 %
pourcentage d'azote : 1,8 % à 2,5 %
pourcentage en poids de groupes acétyle : 0 % à 8 %
pouvoir rotatoire spécifique en solution aqueuse à 20°C :

$$[\alpha]_D^{20} = + 30° \text{ à } + 70°$$

caractérisé en ce que l'on fractionne sur gel de polyacrylamide-agarose comprenant 20 % de polyacrylamide et 2 % d'agarose, une héparine dépolymérisée éventuellement partiellement désulfatée obtenue par action d'une base minérale ou organique sur un ester d'héparine ou d'héparine partiellement désulfatée.

Office européen des brevets

**0133078**

Numéro de la demande

# RAPPORT DE RECHERCHE EUROPEENNE

EP 84 40 1407

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int. Cl. ³) |
|---|---|---|---|
| A | ANALYTICAL BIOCHEMISTRY, vol. 44, 1971, pages 612-622; C.A. McDEVITT et al.: "Gel electrophoresis of proteoglycans and glycosaminoglycans on large-pore composite polyacrylamide-agarose gels" --- | | C 08 B 37/10 |
| D,Y | EP-A-0 040 144 (PHARMINDUSTRIE) * pages 23,24; page 25, tableau B * --- | 1 | |
| Y | EP-A-0 048 231 (AB KABI) * page 3, alinéa 3, point 2; revendications * ----- | 1 | |
| | | | DOMAINES TECHNIQUES RECHERCHES (Int. Cl. ³) |
| | | | C 08 B<br>C 07 H<br>A 61 K |

Le présent rapport de recherche a été établi pour toutes les revendications

| Lieu de la recherche | Date d'achèvement de la recherche | Examinateur |
|---|---|---|
| LA HAYE | 12-10-1984 | LENSEN H.W.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82